# EUROPEAN PATENT APPLICATION

(11) **EP 2 913 080 A2**
(43) Date of publication of application: **02.09.2015**
(21) Application number: 13848516.4
(22) Date of filing: 22.10.2013
(51) Int. Cl.: A61M 25/00, A61M 25/01

(54) **METHOD FOR THE CATHETERIZATION OF THE CORONARY ARTERIES AND CATHETER FOR THE IMPLEMENTATION THEREOF**

(30) Priority: 23.10.2012 RU 2012145031; 27.12.2012 RU 2012157531
(71) Applicant: Osiev, Aleksandr Grigorievitch, Novosibirsk 630090 (RU)
(72) Inventor: Osiev, Aleksandr Grigorievitch, Novosibirsk 630090 (RU)
(74) Representative: Grünecker Patent- und Rechtsanwälte PartG mbB
(86) International application number: PCT/RU2013/000939
(87) International publication number: WO 2014/065714

(57) **Abstract**

What is proposed is a method for the catheterization of the coronary arteries in which a catheter is introduced via the venous system into the right atrium, then passed through the interatrial septum and introduced into the left atrium, followed by the left ventricle, and into the ascending aorta to the opening of the coronary arteries, and also a catheter for the implementation of this method.

## Description

### Field of invention

The invention relates to medicine, namely to cardiology, and is intended for the diagnostics and treatment of heart disease, particularly ischemic heart disease (IHD).

### Background

A pathological condition characterized by a difference between the required blood supply for an organ and the actual blood flow shall be referred to as ischemia. This pathology occurs due to the narrowing of an artery by atherosclerosis, spasm or occlusion with thrombi.

An injury located in the heart artery (coronary circulation failure) leads to development of various forms of the IHD (myocardial infarction, angina pectoris, sudden cardiac death, etc.).

Myocardium is supplied with oxygen and nutrients through the right and left coronary arteries, which extend from the aortic root (gate) directly above the aortic valve cusps. Coronary arteries (i.e. vessels that supply blood to the myocardium) are the only source of blood supply to the myocardium, that is why stenosis (narrowing of the lumen) of these vessels is so critical.

Recovery (reperfusion) of the blood flow in an organ which has suffered from long term acute ischemia, is followed by reperfusion injury in tissue.

In view of heart injury during ischemia and possibility of the reperfusion complications it is desirable to provide means for ant ischemic protection.

In case of acute myocardial infarction the most effective means of therapy is a method of express balloon angioplasty and stenting of the coronary arteries, which allows to significantly reduce mortality and frequency of the major complications from the disease.

On the other hand, accurate diagnostics is essential for prescribing a proper treatment.

One of the modem means for IHD diagnostics is coronary angiography (or coronarography), i.e. examination of the heart vessels (coronary arteries) by injection of a radiopaque substance. Angiography allows visualization of the blood vessels in the heart and assessment of their anatomy, size, lumen, contour. Coronary catheterization allows to determine the condition of the coronary arteries, verify the diagnosis and define indications for endovascular, surgical, or conservative treatment.

Both the diagnostic and treatment methods include catheterization of the heart vessels.

Coronary interventions are traditionally performed by the method of Judkins (Judkins, M.P.) where catheter is inserted through the femoral artery. Alternative access is via the radial artery.

A problem with the current approach is the arterial access itself, which significantly limits the diameter of the catheters used and may be associated with a number of serious complications, such as bleeding, thrombosis, dissection of a large vessel, acute ischemia, and many others, including the death of the patient. Further, in some cases (pulse absence, aortic dissection) catheterization via arterial access is technically impossible.

### Summary of invention

The objective of this invention is overcoming the limitations and complications of traditional coronary intervention and developing an alternative method of access to the coronary arteries, particularly, a catheterization method.

This objective is attained by providing the method for catheterization of coronary arteries of invention, in which a catheter is inserted through the venous system into the right atrium, then passed through interatrial septum into the left atrium and further into the left ventricle and ascending aorta to the coronary ostia. In this method a catheter is used configured in such a way that its distal end, when inserted into ascending aorta, is facing the coronary ostium.

According to one embodiment of the invention, cauterization is performed for the purpose of angiography.

Further, according to the invention, catheters for catheterization of the coronary arteries are provided, the catheters being configured in such a way that the distal end of said catheter is facing the coronary ostium when catheter is inserted into the ascending aorta via the left ventricle.

According to one embodiment of the invention the catheter is designed for angiography.

It is advantageous that the catheter of this invention comprises three integrated portions: a proximal portion, a vertical portion and a horizontal or distal portion, wherein the junction between the proximal portion and the vertical portion (proximal bend) is located within the left ventricle when the catheter is in its operating position.. Further, when the catheter is in its operating position the other end of the vertical portion is located outside the left ventricle in the ascending aorta, and the horizontal portion is located in the ascending aorta and is bended so that the tip of the catheter is positioned at the coronary ostium.

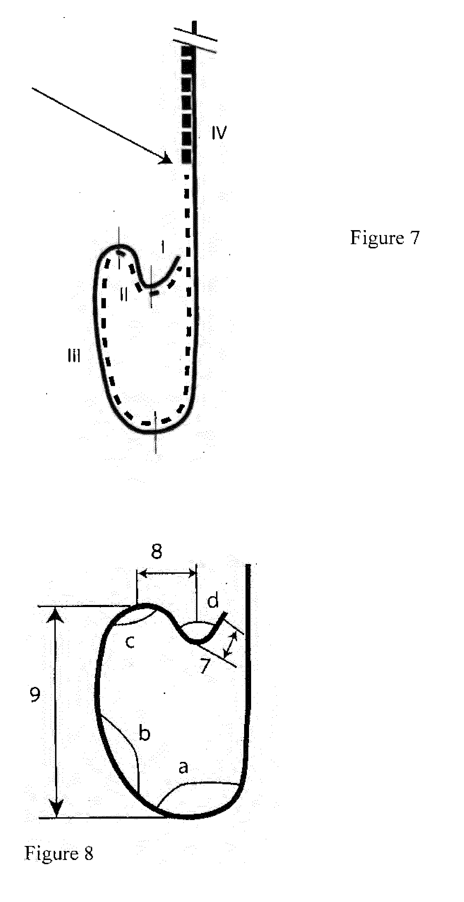

In some embodiments of the invention catheter tips are shaped as shown in Fig. 7 and 8, 10, 12, or 17.

In one embodiment a catheter is provided for the left coronary artery, wherein the horizontal portion of the catheter is bended, and the distance from the tip of the catheter to the central point of the bend of the horizontal portion is 2 to 32 mm, for example 9 mm, the distance from the central point of the bend of the horizontal portion to the central point of the bend between the vertical and horizontal portions of the catheter is 2 to 62 mm, e.g. 16 mm, and the distance from the central point of the bend between the horizontal and vertical portions of the catheter to the central point of the proximal bend is 12 to 200 mm, for example 73 mm, wherein the bend of the horizontal portion and the proximal bend are oriented in the same direction, and positioned on the same side of the vertical portion, while the bend between the vertical and the horizontal portions is oriented in the opposite direction thereto. Further, the bend of the horizontal portion has an angle 10 to 179°, preferably 40 to 110°, more preferably approximately 90° (on the average), the bend between the vertical and horizontal portions has an angle 10 to 179°, preferably 40 to 60°, more preferably about 45° (on the average), the proximal bend has an angle 10 to 179°, preferably 40 to 60°, more preferably about 45° (on the average), and the vertical portion itself may be bent at an angle up to 180°, preferably 40 to 120°, more preferably about 90° (on the average). The catheter has a circular shape, nominally shape of the letter "O." Sizes of the portions correspond to the sizes of relevant chambers in the heart.

In another embodiment a catheter for the left coronary arteria is provided, the catheter having two bends in its horizontal portion, wherein the distance between the tip of catheter and the central point of the first bend of the horizontal portion is 2 to 32 mm, for example 35 mm, the distance from the central point of the first bend of the horizontal portion to the central point of the second bend of the horizontal portion is 2 to 120 mm, for example 29 mm, the distance from the central point of the second bend of the horizontal portion to the central point of the bend between the horizontal and vertical portions of the catheter is 2 to 80 mm, for example 29 mm, and the distance from the central point of the bend between the horizontal and vertical portions of the catheter to the central point of the proximal bend is 2 to 200 mm, for example 38 MM. The first bend of the horizontal portion and the proximal bend are oriented in the opposite directions and positioned on the same side of the vertical portion, while the first bend of the horizontal portion has an angle 10 to 179°, preferably up to 110°, more preferably approximately 90° (on the average), the bend between the horizontal and vertical portions has an angle 10 to 179°, preferably 40 to 110°, more preferably about 90° (on the average), the proximal bend has an angle 10 to 179°, preferably 80 to 110°, more preferably about 90° (on the average), and the vertical portion itself may be bent at an angle up to 180°. The proximal bend is configured in such a way as to form a circular shape, and the horizontal portion together with the vertical portion are configured in the shape close to letter "M." The sizes of the portions correspond to the sizes of relevant chambers in the heart. To ensure access to the coronary artery the catheter tip can be additionally bended.

In one more embodiment a catheter is provided for the right coronary artery, wherein the horizontal portion of the catheter is bended, and the distance between the tip of catheter and the central point of the bend of the horizontal portion is 2 to 32 mm, for example 9 mm, the distance from the catheter tip to the central point of the bend between the horizontal and vertical portions of the catheter is 4 to 80 mm, e.g. 29 mm, and the distance from the central point of the bend between the horizontal and vertical portions of the catheter to the central point of the proximal bend is 6 to 200 mm, for example 41 mm. The bend of the horizontal portion and proximal bend are oriented in the same direction, and positioned on different sides of the vertical portion. The bend of the horizontal portion has an angle 90 to 180°, preferably 90 to 170°, more preferably 110° (on the average), the bend between the vertical and horizontal portions has an angle 10 to 179°, preferably 40 to 110°, more preferably about 90° (on the average), the proximal bend has an angle 10 to 179, preferably 40 to 110° more preferably 70° (on the average), and the vertical portion itself may have two bends at the angle 10 to 180°, preferably 90 to 140°, more preferably about 110° (on the average).

An angle of a bend is an angle defined by two tangent lines. All the bends are even.

The proximal portion of a catheter is the portion closest to the operator. The distal portion is the portion closest to the coronary artery. An end part of the proximal portion of the catheter forms a "proximal bend" at the junction with the "vertical part," while another end part of the proximal portion, in the operating position being located outside the human body, is used for performing surgical manipulations therethrough. The length of the proximal part may be up to 900 mm.

The method of invention is novel because it suggests a new path for leading a catheter to the coronary arteries, never used before neither clinically, nor experimentally. Unlike the conventional method, this method allows antegrade (i.e., in the normal direction of the blood flow) access into the heart arteries through the femoral venous access, left atrium and left ventricle to the aortic root.

The method allows to access coronary arteria in case of contraindications to traditional arterial access.

The method allows to perform coronography and percutaneous coronary interventions without puncture of peripheral arteries, which in some cases is the only possibility to perform endovascular diagnostics or treatment. This method also allows to avoid complications related to arterial access. The method allows to increase efficiency of a treatment by safe escalation of the therapeutic dose while eliminating adverse drug reaction caused by some pharmaceutical preparations (for example, thrombolytic) due to decreased risk of hemorrhage from the puncture point.

The method also allows to perform coronography and/or percutaneous coronary interventions simultaneously with other endovascular procedures performed through the venous access, for example, radiofrequency ablation.

### Brief description of the drawings

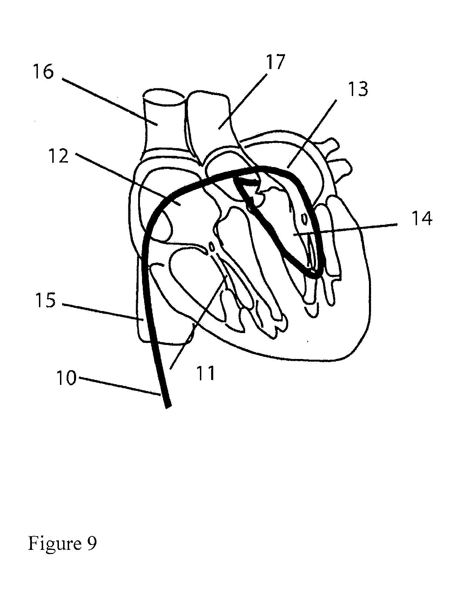

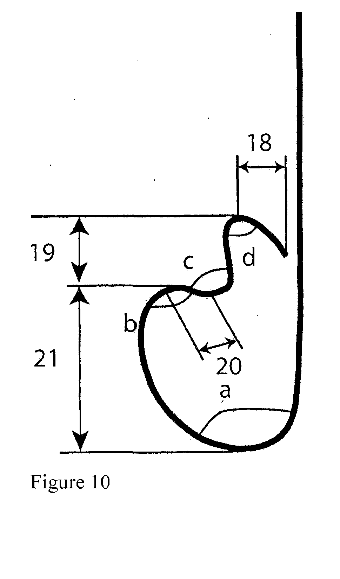

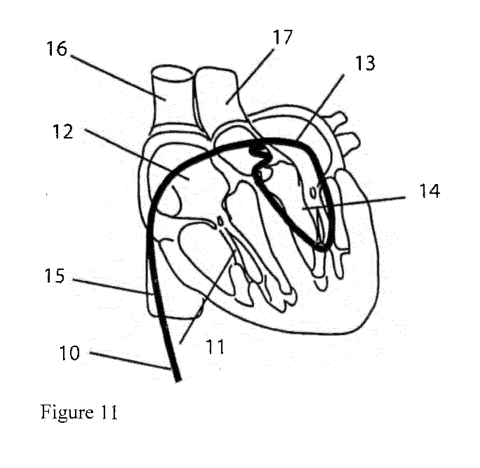

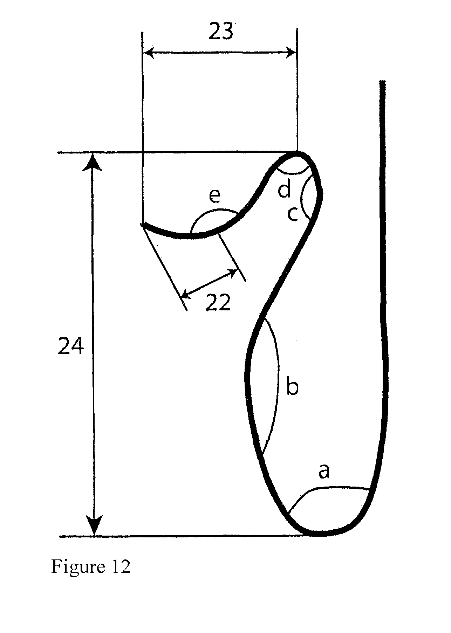

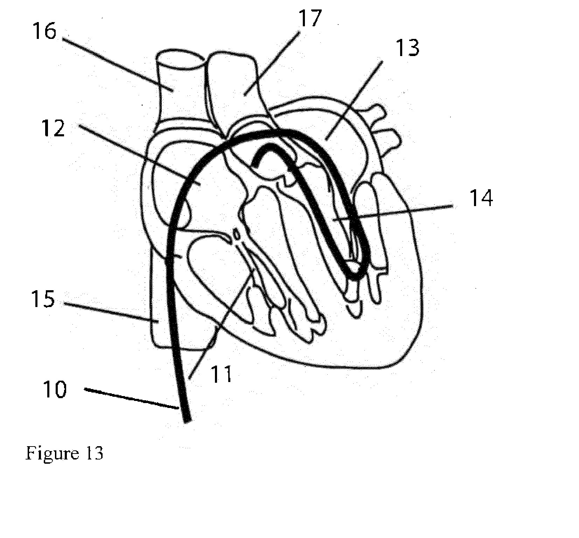

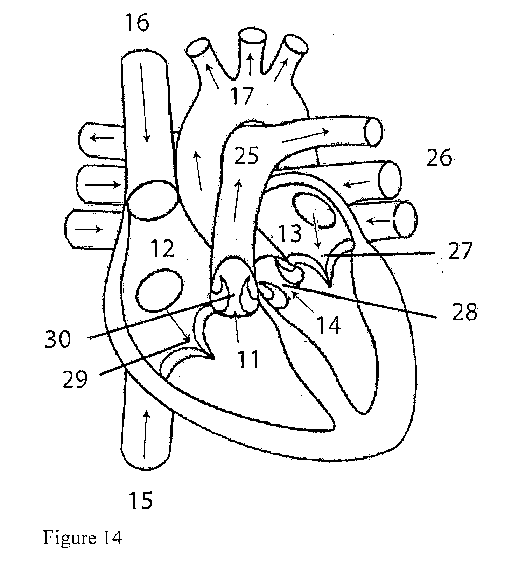

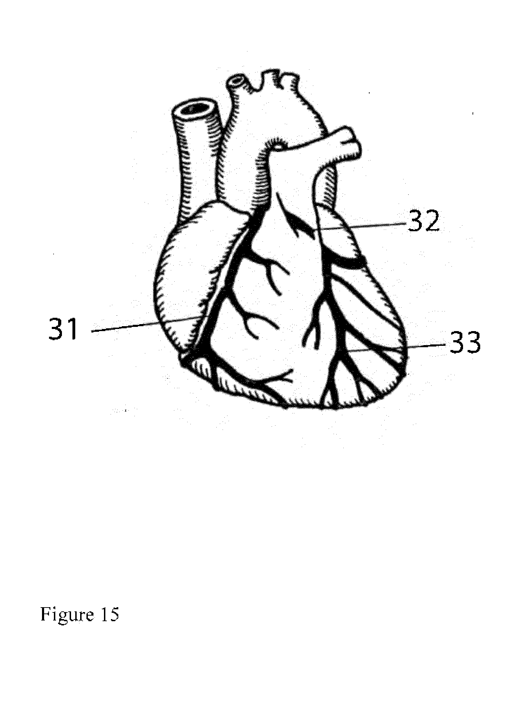

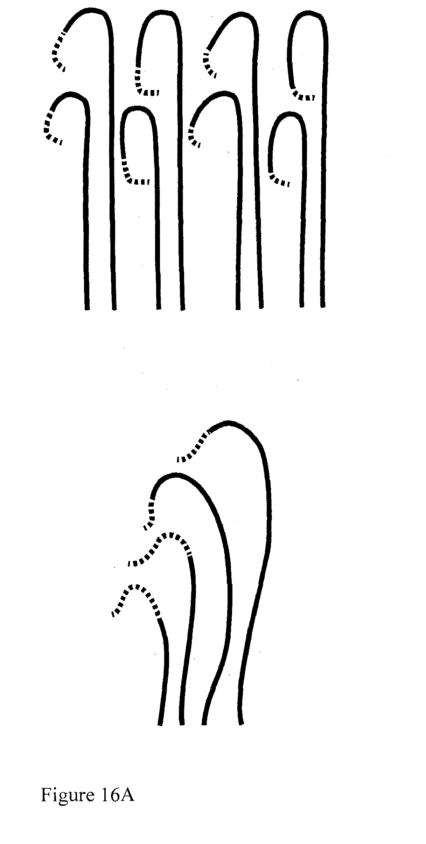

Fig. 1. Guide wire 0.035' is passed from the vena cava inferior into the right atrium, further on through interatrial septum defect into the left atrium, and then into the left ventricle and the ascending and descending aorta.
Fig. 2. Selective coronarography of the left coronary artery.
   1. Supporting catheter
   2. Catheter of embodiment 1
   3. Left coronary artery
Fig. 3. Selective coronarography of the right coronary artery.
   4. Guiding catheter for support
   5. Catheter of embodiment 2
   6. Right coronary artery
Fig. 4. Coronary angiogram of the left coronary artery, transvenous access. Stenosis of the left descending anterior artery (arrow).
Fig. 5. Implantation of a coronary stent, transvenous access.
Fig. 6. Final result after stenting.
Fig. 7. Catheter for the left coronary artery **(OsievO)**
   The catheter of the invention has four bends:
   L-II. Horizontal portion of the catheter (distal portion, including the tip of the catheter).
   III. Vertical portion of the catheter
   IV. Rigid and proximal portion of the catheter up to the middle of the proximal bend.
Fig. 8. Catheter for the left coronary artery **(OsievO).**
   Distances:
   7. from the soft tip to the center of the angle d 2 - 32 mm
   8. from the central portion of the angle d to the center of the angle c 2 - 62 mm
   9. from the central portion of the angle c to the center of the angle a 12 - 200 mm
   Angles
   a. 10 - 179°
   b. 10 - 180°
   c. 10 - 179°
   d. 10 - 179°
Fig. 9. Relative position of the left coronary artery catheter (OsievO), heart, and great vessels.
   10. Catheter
   11. Right ventricle cavity
   12. Right atrium cavity
   13. Left atrium cavity
   14. Left ventricle cavity
   15. Inferior vena cava
   16. Superior vena cava
   17. Ascending aorta
Fig. 10. Catheter for the left coronary artery **(OsievM).** The catheter has four bends.
   Distances:
   18. from the soft tip to the center of the angle d 2 - 32 mm
   19. from the central portion of the angle d to the center of the angle c 2 -120 mm
   20. from the central portion of the angle c to the center of the angle b 2 - 80 mm
   21. from the central portion of the angle c to the center of the angle a 2 -120 mm Angles
      a. 10 - 179°
      b. 10 - 179°
      c. 10 - 179°
      d. 10 - 179°
Fig. 11. Relative position of the left coronary artery catheter **(OsievM),** heart, and great vessels.
   10. Catheter
   11. Right ventricle cavity
   12. Right atrium cavity
   13. Left atrium cavity
   14. Left ventricle cavity
   15. Inferior vena cava
   16. Superior vena cava
   17. Ascending aorta
Fig. 12. Catheter for the right coronary artery **(OsievR).** The catheter has five bends.
   Distances:
   22. from the tip to the center of the angle e 2 - 32 mm
   23. from the soft tip to the center of the angle d 4 - 80 mm
   24. from the central portion of the angle d to the center of the angle a 6 - 220 mm
   Angles
      a. 10 - 179°
      b. 10-180°
      c. 10-180°
      d. 10-179°
      e. 90-180°
Fig. 13. Relative position of the right coronary artery catheter **(OsievR),** heart, and great vessels.
   10. Catheter
   11. Right ventricle cavity
   12. Right atrium cavity
   13. Left atrium cavity
   14. Left ventricle cavity
   15. Inferior vena cava
   16. Superior vena cava
   17. Ascending aorta
Fig. 14. Human heart.
   11. Right ventricle cavity
   12. Right atrium cavity
   13. Left atrium cavity
   14. Left ventricle cavity
   15. Inferior vena cava
   16. Superior vena cava
   17. Ascending aorta
   25. Pulmonary trunk
   26. Pulmonary veins
   27. Mitral valve
   28. Aortic valve
   29. Tricuspid valve
   30. Pulmonary valve
Fig. 15. Coronary arteries.
   31. Right coronary artery
   32. Left coronary artery
   33. Anterior descending artery
Fig. 16A - 16B. Shapes of traditional catheter.

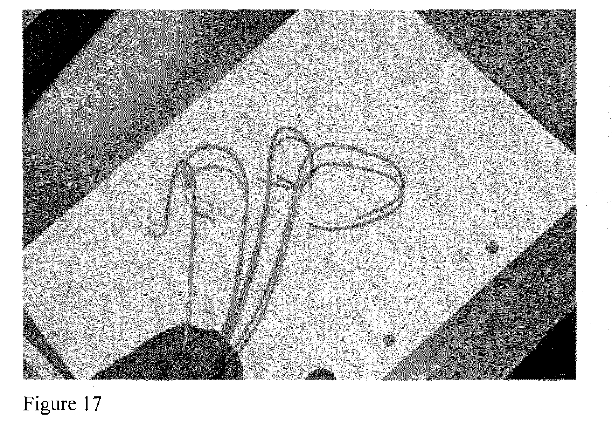

Fig. 17. Catheters according to the invention.

### Detailed description of the invention

Although penetration into the left atrium through the venous access with atrial septal puncture has been proposed in the prior art (US6241728), but this method has never been applied for coronary intervention.

As discovered by the inventor, coronary arteries can be reached through the left atrium (via the venous access with atrial septal puncture) for catheterization and manipulation just by changing the shape of a standard catheter.

Whereas this catheterization technique may seem unobvious to those skilled in the art, the inventor has performed a number of successful operations using this technique, thus proving its feasibility.

In traditional catheterization methods, puncture of arteria (femoral or radial) is performed, and a catheter is inserted through a guide in an artery into ascending aorta, where, due to a suitably selected shape of the catheter tip, the catheter is positioned with its distal end adjacent to the ostium of the coronary artery (left or right).

For conventional catheterization with access to the coronary arteries through peripheral arteries and aorta a range of catheters manufactured on industrial scale is used.

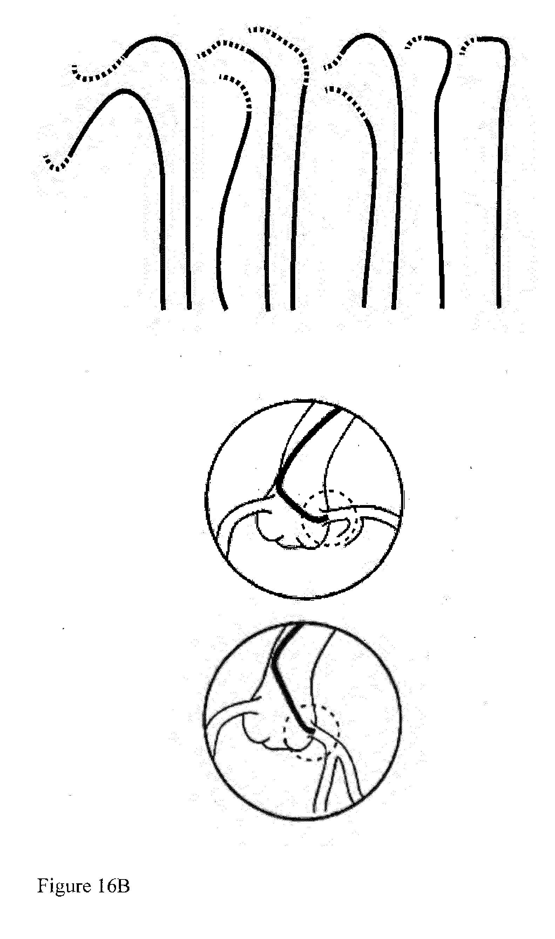

For the purposes of discussion, traditional catheters can be grouped into several categories by the shape of their distal part and their diameter (see Fig. 16). Standard types of diagnostic catheters are Judkins type catheters for the left and right coronary arteries, the catheters having respective bends of varied length (1 to 6 cm) and tips of varied length (standard-length tips up to 2 cm and short-cut tips up to 1 cm), as well as Amplatz type catheters having respective bends of varied bending radii: 1 to 3 cm (also for the left and right coronary arteries). The catheters differ by the angle to which the tip is bent and direction in which the tip is bent, particularly in the horizontal plane.

However, in spite of variety of catheters in terms of architecture, interior and exterior diameters they all are made of radiopaque polymer and have one lumen. All catheters comprise a hollow polymer tube with a length 60 to 200 cm and an outer diameter 3 to 21 F (1 to 7 mm). Until recently these catheters well satisfied the requirements of the professionals practicing in the field of interventional cardiology.

However, none of the existing catheters is adapted for coronary artery catheterization through the venous access because their bends do not correspond to the heart anatomy and do not allow to selectively place the catheter tip at the ostia of the coronary arteries.

A characteristic difference between the catheters of invention and existing diagnostic and guiding catheters are unique shapes of the catheters of invention, defined by bends and angles of catheter terminal portions, allowing unhindered access of the catheter tip to the ostium of the coronary artery. The catheters of invention match with the anatomy of the heart.

A catheter of invention includes rigid portions and soft portions. Its total length be up to 100 cm. A soft portion (0.1-30 cm) extends from the zone pointed with the arrow in Fig. 7 to the distal end of catheter (up to the tip positioned at the ostia of the coronary arteries).

The inventor suggests the following preferable shapes of catheters:
1. Catheter for catheterization of the left coronary artery (Figs. 7-9) characterised in that it has specific bends at the distance 4 to 40 cm from the distal end. The catheters may be referred to as OsievM - 1, OsievM - 2., 3., 3.5.,4.0., 4.5.,
   further up to 10). Also intermediate modifications may exist, for example OsievM 3.75, OsievM 2.24, and others. The catheter is named so due to the form of its portion which resembles the letter "M."
2. Catheter for catheterization of the left coronary artery (Figs. 10-11) characterised in that it has specific bends at the distance 4 to 20 cm from the distal end. The catheters may be referred to as OsievO - 1, OsievO - 2., 3.5., 4.0., 4.5., further up to 10. Also intermediate modifications may exist, for example OsievO 3.75, OsievO 2.24, and others. The catheter is named due to the form of its portion which resembles the letter "O."
3. Catheter for catheterization of the right coronary artery OsievR. R means right (Figs. 12-13) characterised in that it has specific bends at the distance 4 to 20 cm from the distal end. The catheters may be referred to as OsievR - 1., OsievR - 2., 3.0., 3.5., 4.0., 4.5, further up to 10. Also intermediate modifications may exist, for example OsievR 3.75, OsievR 2.24, and others.

The method for catheterization is as follows. Under local anesthesia right or left femoral vein puncture (by Seldinger) is performed. A diagnostic catheter, for example PigTail, is inserted on the diagnostic guide wire, for example 0.35 ", through an installed introducer. The catheter is inserted through inferior vena cava into the right atrium and further through the atrial septal defect (or following a transseptal puncture) into the left atrium, and then through the mitral valve into the left ventricle and through the aortic valve into the aorta. Diagnostic guide wire of standard length is replaced with a diagnostic guide wire 300 mm length. This guide wire is used for guiding a catheter of invention, which is inserted up to aortic sinuses. Further, the diagnostic guide wire is removed and the required manipulation such as selective angiography of the coronary arteries or stenting is performed.

The method for catheterization and catheters as suggested by the inventor were used for coronarography and coronary stenting.

### Coronarography

Examination of the coronary arteries were performed in patients with atrial septal defect (ASD).

The examined group consisted of patients with secondary ISD aged 45 with indications for coronary angiography. Each patient gave a voluntary written consent to undergo this method. To be sure there are no clots in the cavity of the left atrial appendage all patients were subjected to transesophageal echocardiography.

Clinical characteristics of patients are shown in Table 1.

| Characteristics | N=21 |
|---|---|
| Average age, years. | 50.85±5.96 |
| Female, n(%) | 17 (80.9) |
| Arterial hypertension, n (%) | 16 (76.2) |
| Hyperlipidemia, n (%) | 15 (71.4) |
| Diabetes, n (%) | 2 (9.5) |
| Pulmonary hypertension P>35 mm Hg, n (%) | 19 (90.5) |
| CHEF I-II, n (%) | 21 (100) |

All patients were subjected to coronary angiography according to the following method.

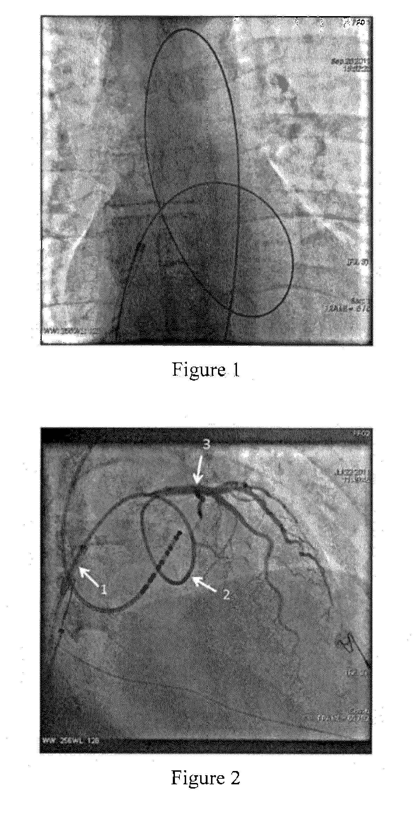

Catheterization method:
Step 1: Femoral vein puncture (by Seldinger) for installation of an introducer.
Step 2: Through the installed introducer a diagnostic catheter, for example, JR 3.5, on diagnostic guide wire, for example 0.35 " J type 300 cm, is inserted into the right atrium 5 through the inferior vena cava in the direction of the venous blood flow. The diagnostic guide wire is subsequently used for preparation to transseptal puncture.
Step 3: Atrial septum puncture. This step may be omitted in case of opened foramen oval or defect of the septum.
Step 4: Insertion of a diagnostic guide wire, for example, 0.35" J type 300 cm, into the left atrium.
Step 5: Insertion of a catheter, for example JL 4.0, and a guide wire 15 such as 0.35" J type, see 300, through the left atrium and the left ventricle into the aorta, bypassing aortic and mitral valves (Fig. 1).
Step 6: Guide wire is left in the aorta (preferably up to descending part) and catheter is replaced with a new catheter adapted to access to the ostium of the coronary artery.
Step 7: Coronary artery catheterization and coronary angiography (Fig. 2 -3).

Catheters adapted to access to the ostia of the coronary arteries were made by the inventor (Fig. 17). Several types of catheters were used (Fig. 7 - 13, 17).

Coronarography shall be performed in the common angiographic projections. For this purpose a contrast agent (Optiray (Ioversol) 350 by Tyco, USA was administered through coronary catheter lumen with Luer type syringe or infusion pump by ACIST, USA.

Radiological control and digital recording of the results was performed using Innova 4100 roentgen graphic angiographic device by GE, USA.

### Results.

Selective coronarography was successfully performed in all patients. However, when passing the left ventricle dysrhythmia was detected in each case. No other complications were detected. Main parameters of interference are shown in Table 2.

| Parameter | Venous access |
|---|---|
| The average volume of contrast agent administered, ml. | 82.31+31.41 |
| Average time of the procedure, min | 101.15±33.29 |
| Average time of the fluoroscopy, min | 23.03±9.59 |

### Stenting

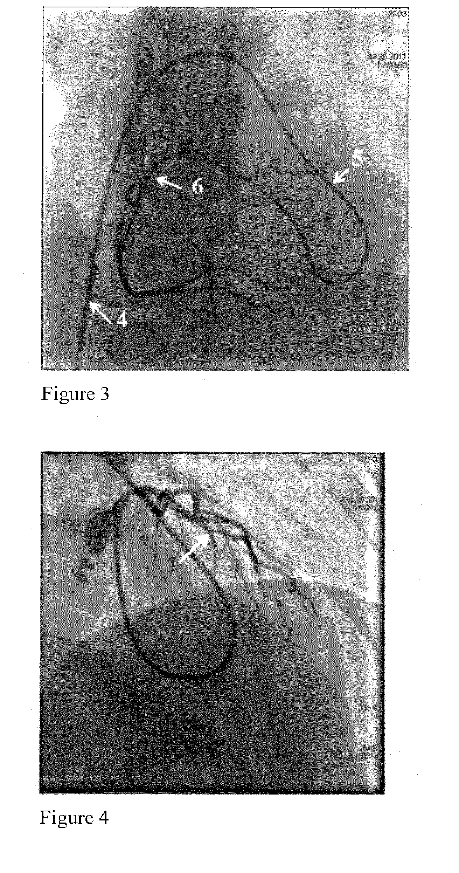

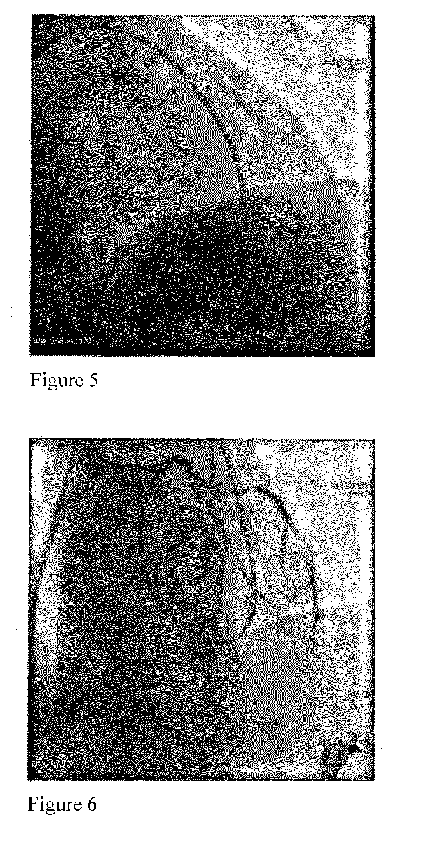

The first clinical example of successful implantation of coronary stents is the patient with atrial septal defect diagnosed by echocardiography. Coronarography: surgically significant stenosis of the anterior descending artery (Fig. 4). Positive treadmill test. Angioplasty with stenting of the target artery is performed through antegrade venous access as disclosed here (Fig. 5), with excellent angiographic result (Fig. 6).

The method of invention is highly useful in certain clinical situations, and sometimes is the only possible method to perform intervention. In practice cardiac surgeons face bilateral occlusion of the iliac and/or arteria subclavian, various types and configurations of aorta, and brachiocephalic arteries. In such situations venous access may appear to be the best, and sometimes the only possible option to perform endovascular coronary intervention. In clinical practice such situations occur ever so often, and their number will ever grow. It shall be noted that this approach can also be used for non-coronary interventions. Further, there is an experience of using this access for stenting of brachiocephalic arteries.

The inventor has demonstrated the feasibility and safety of the claimed method of percutaneous coronary interventions for diagnostics and treatment of heart diseases.

## Claims

1. Method for catheterization of the coronary arteries, wherein a catheter is inserted through the venous system into the right atrium, then passed through interatrial septum into the left atrium and further into the left ventricle and ascending aorta to the coronary ostia.

2. Method according to claim 1, in which is used catheter distal end of which after insertion into ascending aorta is positioned opposite coronary ostium.

3. Method according to claim 1, where catheterization is performed for the purpose of angiography.

4. Catheter for catheterization of coronary arteries, the catheter being configured in such a way that the distal end of said catheter is facing the coronary ostium when the catheter is inserted into ascending aorta via the left ventricle.

5. Catheter according to claim 4 for use in angiography.

6. Catheter according to claim 4 is **characterized in that** it comprises three integrated portions: a proximal portion, a vertical portion and a horizontal or distal portion, wherein the junction between the proximal portion and the vertical portion (proximal bend) is located within the left ventricle when the catheter is in its operating position, the other end of the vertical portion is located outside the left ventricle in the ascending aorta when the catheter is in its operating position, and the horizontal portion is located in the ascending aorta and is bended so that the tip of the catheter is positioned at the coronary ostium.

7. Catheter according to claim 4, the catheter having a tip shaped as shown in Figs. 7 and 8, 10, 12, or 17.

8. Catheter according to claim 6, used for the left coronary artery, **characterized in that** the horizontal portion of the catheter is bended, and the distance from the tip of the catheter to the central point of the bend of the horizontal portion is 2 to 32 mm, the distance from said central point of the bend of the horizontal portion to the central point of the bend between the vertical and horizontal portions of the catheter is 2 to 62 mm, and the distance from said central point of the bend between the horizontal and vertical portions of the catheter to the central point of the proximal bend is 12 to 200 mm, wherein the bend of the horizontal portion and the proximal bend are oriented in the same direction, and positioned on the same side of the vertical portion, and the bend between the vertical and the horizontal portions is oriented in the opposite direction thereto, while the bend of the horizontal portion has an angle 10 to 179°, the bend between the horizontal and vertical portions has an angle 10 to 179 °, the proximal bend has an angle 10 to 179°, and the vertical portion itself may be bent at an angle up to 180°.

9. Catheter according to claim 6, used for the left coronary artery, **characterized in that** the horizontal portion of the catheter is configured with two bends, and the distance between the tip of catheter and the central point of the first bend of the horizontal portion is 2 to 32 mm, the distance from the central point of the first bend of the horizontal portion to the central point of the second bend of the horizontal portion is 2 to 120 mm, the distance from the central point of the second bend of the horizontal portion to the central point of the bend between the horizontal and vertical portions of the catheter is 2 to 80 mm, and the distance from said central point of the bend between the horizontal and vertical portions of the catheter to the central point of the proximal bend is 2 to 200 mm, wherein the first bend of the horizontal portion and the proximal bend are oriented in opposite directions and positioned on the same side of the vertical portion, the first bend of the horizontal portion having an angle 10 to 179°, the second bend of the horizontal portion having an angle 10 to 179°, the bend between the horizontal and vertical portions having an angle 10 to 179°, the proximal bend having an angle 10 to 179°, and the vertical portion itself may be bent at an angle up to 180°.

10. Catheter according to claim 6, used for the right coronary artery, **characterized in that** the horizontal portion of the catheter is configured with a bend, wherein the distance between the tip of the catheter and the central point of the bend of the horizontal portion is 2 to 32 mm, the distance from said tip of the catheter to the central point of the bend between the horizontal and vertical portions of the catheter is 4 to 80 mm, and the distance from said central point of the bend between the horizontal and vertical portions of the catheter to the central point of the proximal bend is 6 to 220 mm, wherein the bend of the horizontal portion and proximal bend are oriented in the same direction and positioned on the different sides of the vertical portion, the bend of the horizontal portion has an angle 90 to 180°, the bend between the horizontal and vertical portions has an angle 10 to 179°, the proximal bend has an angle 10 to 179°, and the vertical portion itself may have two bens with the angle 10° to 180°.
